# EUROPÄISCHE PATENTSCHRIFT

(11) **EP 1 224 927 B1**
(45) Veröffentlichungstag und Bekanntmachung des Hinweises auf die Patenterteilung: **27.12.2006**
(21) Anmeldenummer: 02001025.2
(22) Anmeldetag: 17.01.2002
(51) Int. Cl.: A61Q 5/10, A61K 8/34, A61K 8/41

(54) **Haarfärbemittel enthaltend direktziehende Farbstoffe, quaternäre Ammoniumverbindungen und kationische Polymere**
Hair dye composition comprising direct dyes, quaternary ammonium compounds and cationic polymers
Composition de teinture pour fibres kératiniques comprenant un colorant direct, un composé d'ammonium quaternaire et un polymère cationique

(30) Priorität: 17.01.2001 DE 10101946
(43) Veröffentlichungstag der Anmeldung: 24.07.2002
(73) Patentinhaber: KPSS-Kao Professional Salon Services GmbH, 64297 Darmstadt (DE)
(72) Erfinder: Grit, Mustafa, Dr., 64579 Gernsheim (DE)

(56) Entgegenhaltungen:
- EP-A- 0 470 381
- EP-A- 0 503 507
- DE-A- 2 151 131
- DE-A- 19 735 851
- US-A- 4 820 308

## Beschreibung

Die Erfindung betrifft ein gelförmiges Haarfärbemittel auf wäßriger Basis, das mindestens einen direktziehenden Haarfarbstoff enthält.
Derartige Haarfärbemittel sind seit langem bekannt.
Sie enthalten meistens mehrere anionische oder kationische direktziehende Farbstoffe und bedürfen, im Gegensatz zu den permanenten Haarfärbemitteln auf Basis von Oxidationsfarbstoffvorprodukten, keiner vorherigen Entwicklung mit Oxidationsmitteln.
Diese direktfärbenden Zusammensetzungen werden entweder zusammen mit oberflächenaktiven Mitteln als sogenannte Tönungsshampoos appliziert, oder, wie die erfindungsgemäßen Zusammensetzungen, als Lotionen, Emulsionen oder verdickte Lösungen, d.h., Gele, auf das Haar aufgebracht.
Haarfärbegele müssen einerseits so viskos sein, daß sie auf dem Haar verbleiben, um eine ausreichende Färbewirkung zu gewährleisten, müssen sich aber andererseits nach vollendeter Färbung wieder vollständig und leicht aus dem Haar ausspülen lassen.
Derartige Zusammensetzungen enthalten also in der Regel ein Verdickungsmittel.
Es ist wünschenswert, daß diese Gele auch transparent, d.h. optisch klar oder zumindest transluzent, d.h. durchscheinend sind, insbesondere, wenn sie in ein transparentes Verhältnis abgepackt sind.
Die Erfüllung dieser Anforderungen bedarf einer sorgfältigen Abstimmung aller Bestandteile.
Die Erfindung geht daher von der Aufgabenstellung aus, ein Haarfärbemittel auf wäßriger Basis, das als transparentes oder transluzentes Gel auf das Haar aufgebracht werden kann, zu schaffen, das optimale rheologische Eigenschaften aufweist, über längere Zeit stabil bleibt, sowohl mit anionischen als auch kationischen direktziehenden Haarfarbstoffen ausgezeichnete färberische Eigenschaften zeigt, und bei der Abpackung in transparente Verpackungsmaterialien ein ästethisch ansprechendes Erscheinungsbild ergibt.

Die Lösung dieser Aufgabe besteht in einem transparenten bzw. transluzenten gelförmigen Mittel, das
a) 0,001 bis 5 Gew.-% mindestens eines direktziehenden Haarfarbstoffs;
b) 0,1 bis 10 Gew.-% mindestens einer langkettigen quaternären Ammoniumverbindung gemäß Anspruch 1.
c) 0,1 bis 10 Gew.-% mindestens eines kationischen,Polymeren;
d) 0,1 bis 5 Gew.-% mindestens eines nichtionischen und/oder amphoteren bzw. zwitterionischen Polymeren;
e) 15 bis 50 Gew.-% mindestens eines niederen Alkohols, ausgewählt aus Ethanol, n-Propanol und/oder Isopropylalkohol; und
f) Wasser enthält, und eine Viskosität von 1000 bis 50000, vorzugsweise 1500 bis 25000 mPa.s bei 25°C, gemessen im Brookfield-Viskosimeter (RVT), Spindel Nr. 5, aufweist.

Vorzugsweise ist dieses Mittel in einem transparenten Behältnis abgepackt, um sein ästhetisch ansprechendes Aussehen voll zur Geltung zu bringen.

Der Anteil der direktziehenden Farbstoffe in den erfindungsgemäßen Zusammensetzungen ist variabel und liegt zwischen 0,001 bis 5, vorzugsweise 0,01 bis 2,5, insbesondere 0,1 bis 1 Gew.-% des Mittels.
Als direktziehende Haarfarbstoffe können im Prinzip alle für diesen Zweck vorgeschlagenen kationischen Farbstoffe verwendet werden.
Bevorzugt sind die sogenannten "Arianor"-Farbstoffe; vgl. K. Schrader, "Grundlagen und Rezepturen der Kosmetika", 2. Aufl. (1989), S. 811.

Besonders geeignete basische (kationische) Farbstoffe sind:

| | |
|---|---|
| Basic Blue 6, | C.I.-No. 51,175; |
| Basic Blue 7, | C.I.-No. 42,595; |
| Basic Blue 9, | C.I.-No. 52,015; |
| Basic Blue 26, | C.I.-No. 44,045; |
| Basic Blue 41, | C.I.-No. 11,154; |
| Basic Blue 99, | C.I.-No. 56,059; |
| Basic Brown 4, | C.I.-No. 21,010; |
| Basic Brown 16, | C.I.-No. 12,250; |
| Basic Brown 17, | C.I.-No. 12,251; |
| Natural Brown 7, | C.I.-No. 75,500; |
| Basic Green 1, | C.I.-No. 42,040; |
| Basic Red 2, | C.I.-No. 50,240; |
| Basic Red 22, | C.I.-No. 11,055; |
| Basic Red 51, | |
| Basic Red 76, | C.I.-No. 12,245; |
| Basic Violet 1, | C.I.-No. 42,535; |
| Basic Violet 3, | C.I.-No. 42,555; |
| Basic Violet 10, | C.I.-No. 45,170; |
| Basic Violet 14, | C.I.-No. 42,510; |
| Basic Yellow 57, | C.I.-No. 12,719; |
| Basic Yellow 87 und | |
| Basic Orange 31. | |

Selbstverständlich ist auch die Verwendung entsprechender direktziehender Pflanzenfarbstoffe oder auch anionischer (saurer) direktziehender Haarfarbstoffe möglich.

Diese werden üblicherweise ebenfalls in einer Menge von etwa 0,005 bis etwa 5, vorzugsweise etwa 0,05 bis etwa 2,5, insbesondere etwa 0,1 bis etwa 1 Gew.-%, berechnet auf die Gesamtzusammensetzung des Mittels,das als Lösung, Dispersion, Emulsion, Gel oder Aerosolpräparat zur direkten Anwendung vorliegt, eingesetzt.

Als geeignete anionische Farbstoffe können beispielsweise Verwendung finden:

| | |
|---|---|
| Acid Black 1, | C.I.-No. 20,470; |
| Acid Blue 1, | C.I.-No. 42,045; |
| Food Blue 5, | C.I.-No. 42,051; |
| Acid Blue 9, | C.I.-No. 42,090; |
| Acid Blue 74, | C.I.-No. 73,015; |
| Acid Red 18, | C.I.-No. 16,255; |
| Acid Red 27, | C.I.-No. 16,185; |
| Acid Red 87, | C.l.-No. 45,380; |
| Acid Red 92, | C.I.-No. 45,410; |
| Acid Orange 7, | C.I.-No. 15,510; |
| Acid Violet 43, | C.l.-No. 60,730; |
| Acid Yellow 1, | C.I.-No. 10,316; |
| Acid Yellow 23, | C.l.-No. 19,140; |
| Acid Yellow 3, | C.I-No. 47,005; |
| Food Yellow No. 8, | C.I.-No. 14,270; |
| D&C Brown No. 1, | C.I.-No. 20,170 |
| D&C Green No. 5, | C.I.-No. 61,570; |
| D&C Orange No. 4, | C.I.-No. 15,510; |
| D&C Orange No. 10, | C.I.-No 45,425:1; |
| D&C Orange No. 11, | C.I.-No. 45,425; |
| D&C Red No. 21, | C.I.-No. 45,380:2; |
| D&C Red No. 27, | C.I.-No. 45,410:1; |
| D&C Red No. 33, | C.I.-No. 17,200; |
| D&C Yellow No. 7, | C.I.-No. 45,350:1; |
| D&C Yellow No. 8, | C.I.-No. 45,350; |
| FD&C Red No. 4, | C.I.-No. 14,700; |
| FD&C Yellow No. 6, | C.I.-No. 15,985. |

Auch pflanzliche Farbstoffe können allein oder in Kombination mit synthetischen Direktziehern Verwendung finden, beispielsweise Henna (rot oder schwarz), Alkannawurzel, Laccainsäure (Stocklack), Indigo, Blauholzpulver, Krappwurzel- und Rhabarberwurzelpulver, etc.

Das erfindungsgemäße Haarfärbemittel kann auch oberflächenaktive Substanzen enthalten, obwohl es sich um kein Tönungsshampoo handelt.
Solche können anionisch, nichtionisch, kationisch oder amphoter bzw. zwitterionisch sein.

Bevorzugt sind nichtionische und amphotere bzw. zwitterionische Tenside in einer Menge zwischen etwa 0,5 und etwa 5 Gew.-%, berechnet auf die zusammensetzung.

Geeignete nichtionische Tenside sind Verbindungen aus der Klasse der Alkylpolyglucoside mit der allgemeinen Formel

R-0-(CH₂CH₂0)ₙ-Zₓ,

worin R eine Alkylgruppe mit 8 bis 20, vorzugsweise 10 bis 14 Kohlenstoffatomen, Zₓ einen Saccharidrest mit 5 bis 6 Kohlenstoffatomen, n, eine Zahl von 0 bis 10, und x eine Zahl zwischen 1 und 5, vorzugsweise 1,1 bis 2,5 bedeuten.
Weitere geeignete nichtionische Tenside in den erfindungsgemäßen Mitteln sind C₁₀-C₂₂-Fettalkoholethoxylate.
Besonders geeignete C₁₀-C₂₂-Fettalkoholether sind die unter den Trivialnamen "Laureth", "Myristeth", "Oleth", Ceteth", "Deceth", "Steareth" und "Ceteareth" nach der CTFA-Nomenklatur mit Anfügung der Zahl der Ethylenoxid-Moleküle bezeichneten Alkylpolyglykolether, z.B. "Laureth-16":
Der durchschnittliche Ethoxylierungsgrad liegt dabei zwischen etwa 2,5 und etwa 25, vorzugsweise etwa 10 und etwa 20.

Andere zusätzlich mitverwendbare nichtionische Tenside sind z.B. die verschiedenen Sorbitanester, wie Polyethylenglykolsorbitanstearinsäureester, Fettsäurepolyglykolester oder auch Mischkondensate aus Ethylenoxid und Propylenoxid, wie sie beispielsweise unter der Handelsbezeichnung "Pluronics^{®}" im Verkehr sind.

Weitere zusätzlich einsetzbare Tenside sind Aminoxide.
Solche Aminoxide gehören seit langem zum Stand der Technik, beispielsweise C₁₂-C₁₈-Alkyldimethylaminoxide wie Lauryldimethylaminoxid, C₁₂-C₁₈-Alkylamidopropyl- oder ethylaminoxide, C₁₂-C₁₈-Alkyldi(hydroxyethyl)-oder-(hydroxypropyl)aminoxide, oder auch Aminoxide mit Ethylenoxid- und/oder Propylenoxidgruppen in der Alkylkette.
Geeignete Aminoxide sind beispielsweise unter den Bezeichnungen "Ammonyx^{®}", "Aromox^{®}", oder "Genaminox^{®}" im Handel.

Weitere fakultative Tensidbestandteile sind Fettsäuremono- und dialkanolamide, wie Cocosfettsäuremonoethanolamid und Myristinfettsäuremonoisopropanolamid.

Geeignete amphotere bzw. zwitterionische Tenside sind insbesondere die verschiedenen bekannten Betaine wie Fettsäureamidoalkylbetaine und Sulfobetaine, beispielsweise Laurylhydroxysulfobetain; auch langkettige Alkylaminosäuren wie Cocoaminoacetat, Cocoaminopropionat und Natriumcocoamphopropionat und -acetat haben sich als geeignet erwiesen.
In einzelnen können Betaine der Struktur 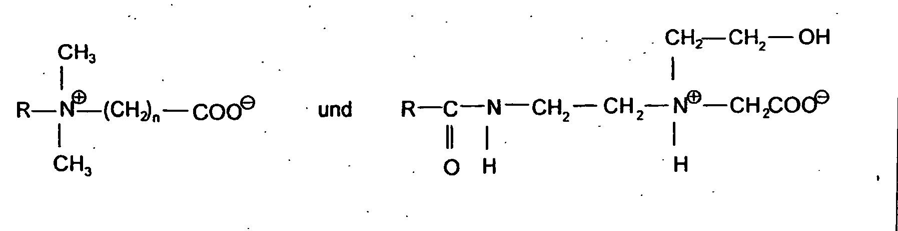 wobei R eine C₈-C₁₈-Alkylgruppe und n 1 bis 3 bedeuten,
Sulfobetaine der Struktur 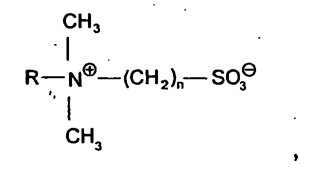 wobei R eine C₈-C₁₈-Alkylgruppe und n 1 bis 3 bedeuten,
und Amidoalkylbetaine der Struktur 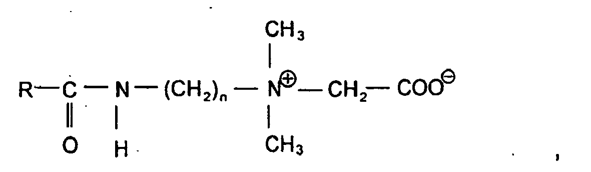 wobei R eine C₈-C₁₈-Alkylgruppe und n 1 bis 3 bedeuten, verwendet werden.
Bevorzugt sind Fettsäureamidoalkylbetaine, insbesondere Cocoamidopropylbetain und Cocoamphoacetat und-propionat, insbesondere deren Natriumsalze.
Besonders bevorzugt sind Gemische aus Cocoamidopropylbetain und Cocoamphoacetat, insbesondere im Gewichtsverhältnis 3:1 bis 1:3, vor allem 2:1 bis 1:1.

Die langkettigen quaternären Ammoniumverindungen im Sinne der Erfindung sind langkettige quaternäre Ammoniumverbindungen, die allein oder im Gemisch miteinander eingesetzt werden können, wie Cetyltrimethylammoniumchlorid, Dimethyldiethylammoniumchlorid, Trimethylcetylammoniumbromid, Stearyltrimethylammoniumchlorid, Dimethylstearylbenzylammoniumchlorid, Benzyltetradecyldimethylammoniumchlorid, Dimethyldihydriertes-Talgammoniumchlorid, Laurylpyridiniumchlorid, Lauryldimethylbenzylammoniumchlorid, Behenyltrimethylammoniumchlorid, Lauryltrimethylammoniumchlorid, Tris-(oligooxyethyl)alkylammoniumphosphat, Cetylpyridiniumchlorid, etc. Gut geeignet sind auch die in der EP-A 472 107 geoffenbarten quaternären Ammoniumsalze.
Weitere geeignete langkettige Ammoniumverbindungen sind Esterquats der allgemeinen Formel (I) 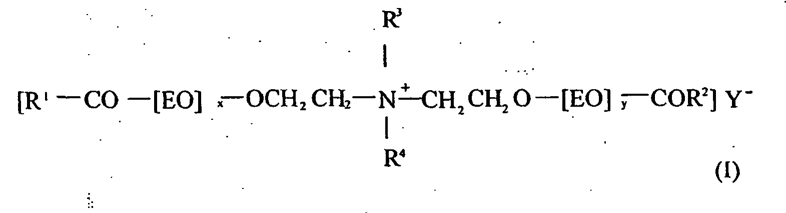 in der R¹ und R² für eine gegebenenfalls hydroxysubstituierte C₈-C₂₂-Alkyl- oder Alkenylgruppe, R³ und R⁴ für eine C₁-C₃-Alkylgruppe oder eine Gruppe -CH₂-CH₂-O-[EO]_{z}H sowie x,y und z für 0 bis 5 und Y⁻ für ein Anion stehen.
Eine besonders bevorzugte Verbindung der Formel I ist im Rahmen der Erfindung eine solche, in der die Reste R¹ und R² jeweils eine Oleylgruppe oder eine C₁₂-C₁₈-Alkylgruppe, der Rest R³ eine Methylgruppe und der Rest R⁴ eine Gruppe -CH₂-CH₂-O-[EO]_{z}-H bedeuten.
Das Anion Y⁻ ist vorzugsweise ein Halogenid wie Cl⁻ oder Br⁻, ein niederes Alkylsulfat, z.B. Methosulfat und Ethosulfat, oder ein Alkylphosphat, jedoch können selbstverständlich auch andere Reste eingesetzt werden.

Diese Verbindungen sind bekannt und beispielsweise unter den Handelsnamen "Schercoquat^{R}", "Dehyquart^{R}F30" und "Tetranyl^{R}" im Handel.
Der Einsatz dieser "Esterquats" in Haarpflegemitteln ist an sich bekannt und beispielsweise in der WO-A 93/10748, der WO-A 92/06899 und der WO-A 94/16677 beschrieben.

Geeignet sind auch Amidoquats der allgemeinen Formel (II) 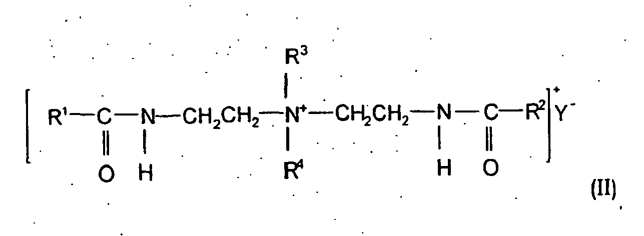 in der R¹ und R² jeweils für eine gegebenenfalls hydroxysubstituierte C₈-C₂₂-Alkyl- oder Alkenylgruppe, R³ und R⁴ für eine C₁-C₃-Alkylgruppe oder eine Gruppe-CH₂-CH₂-O-[EO]ₓ-H, sowie x für 0 bis 5, Y⁻ für ein Anion stehen.

Der Anteil an diesen quaternären Ammoniumverbindungen liegt bei 0,1 bis 10, vorzugsweise etwa 0,25 bis 7,5, insbesondere etwa 0,5 bis 5 Gew.-% des Mittels.

Bevorzugte kationische Polymere sind neben den altbekannten quaternären Cellulosederivaten des Typs "Polymer^{R} JR" insbesondere quaternisierte Homo- oder Copolymere des Dimethyldiallylammoniumchlorids, wie sie unter dem Handelsnamen "Merquat^{R}" im Handel sind, quaternäre Vinylpyrrolidon-Copolymere, insbesondere mit Dialkylaminoalkyl(meth)acrylaten, wie sie unter dem Namen "Gafquat^{R}" bekannt sind, sonstige quaternäre Dimethyl- oder Diethylamino-C₂-C₃-alkyl(meth)acrylat-Copolymere, Copolymerisate aus Vinylpyrrolidon und Vinylimidazoliniummethochlorid, die unter dem Handelsnamen "Luviquat^{R}" angeboten werden, Polyamino-Polyamid-Derivate, beispielsweise Copolymere von Adipinsäure-Dimethylaminohydroxypropyldiethylentriamin, wie sie unter dem Handelsnamen "Cartaretine^{R}F" vertrieben werden, sowie auch bisquaternäre langkettige Ammoniumverbindungen der in der US-PS 4 157 388 beschriebenen Harnstoff-Struktur, die unter dem Handelsnamen "Mirapol A 15" IM Handel sind.

Verwiesen wird in diesem Zusammenhang auch auf die in den Osen 25 21 960, 28 11 010, 30 44 738 und 32 17 059 genannten kationaktiven polymeren sowie die in der EP-A 337 354 auf den Seiten 3 bis 7 beschriebenen Produkte.
Es können auch Mischungen aus verschiedenen kationischen Polymeren eingesetzt werden.

Geeignete kationische Polymere, insbesondere im Gemisch mit nichtionischen, amphoteren bzw. zwitterionischen und/oder anionischen Polymeren, sind auch die aus der EP 640 643 A1 bekannten Organopolysiloxan/Poly-(N-acylalkylenimin)-Copolymerisate.
Grundsätzlich geeignet sind die unter der Bezeichnung "Polyquaternium" im International Cosmetic Ingredient Dictionary der CTFA aufgeführten kationischen Polymeren.
Der bevorzugte Anteil eines solchen kationischen Polymeren liegt bei 0,05 bis 7,5, vorzugsweise 0,1 bis 5, insbesondere 0,25 bis 2,5 Gew.-% des Mittels.

Als amphotere bzw. zwitterionische Polymere, die zum Einsatz gelangen können, seien insbesondere Copolymerisate aus N-Octylacrylamid, (Meth)Acrylsäure und tert.-Butylaminoethylmethacrylat com Typ "Amphomer^{R}", Copolymerisate aus Methacryloylethylbetain und Alkylmethacrylaten vom Typ "Yukaformer^{R}", z.B. das Butylmethacrylat-Copolymere "Yukaformer^{R}Am75"; Copolymerisate aus Carboxylgruppen und Sulfongruppen enthaltenden Monomeren, z.B. (Meth)Acrylsäure und Itaconsäure, mit basischen Gruppen, insbesondere Aminogruppen enthaltenden Monomeren wie Mono- bzw. Dialkylaminoalkyl(meth)acrylaten bzw. Mono- bzw. Dialkalaminoalkyl(meth)acrylamiden; Copolymere aus N-Octylacrylamid, Methylmethacrylat, Hydroxypropylmethacrylat, N-tert.-Butylaminoethylmethacrylat und Acrylsäure sowie die aus der US-A 3,927,199 bekannten Copolymeren genannt.

Geeignete nichtionische Polymere, die statt dessen oder in Kombination mit den zwitterionischen bzw. amphoteren Polymeren eingesetzt werden können, sind vorzugsweise alkohol- bzw. wasserlösliche Vinylpyrrolidon-Polymere wie ein Vinylpyrrolidon-Homo- oder -Copolymerisat, insbesondere mit Vinylacetat.
Geeignete Vinylpyrrolidon-Polymere sind z.B. die unter dem Handelsnamen "Luviskol^{R}" bekannten Produkte, beispielsweise die Homopolymerisate "Luviskol^{R} K30,K60 und K90", sowie die alkohol- bzw. wasserlöslichen Copolymerisate aus Vinylpyrrolidon und Vinylacetat, die unter dem Handelsnamen "Luviskol^{R}VA55 bzw. VA64" von der BASF AF vertrieben werden.
Weitere geeignete nichtionische Polymere sind Vinylpyrrolidon/Vinylacetat/Vinylpropionat-Copolymere wie "Luviskol^{R}VAP343^{R}", Vinylpyrrolidon/(Meth)-Acrylsäureester-Copolymere sowie Chitosan-Derivate.

Der Anteil an nichtionischen und/oder amphoteren bzw. zwitterionischen Polymeren in den erfindungsgemäßen Mitteln liegt bei 0,05 bis 5, vorzugsweise 0,1 bis 3,5, insbesondere 0,25 bis 2,5 Gew.-% der Gesamtzusammensetzung.

Der Anteil an Ethanol, n-Propanol oder Isopropylalkohol beträgt 15 bis 50, insbesondere etwa 20 bis 40 Gew.-% des Mittels.

Weitere Lösungsmittel und Penetrationsvermittler wie 1,2- und 1,3-Propandiol, 1-Methoxypropanol(-2), 1-Ethoxypropanol(-2), 1,3- und 1,4-Butandiol, Glycerin, Benzylalkohol, Benzyloxyethanol, Propylencarbonat, Diethylenglykol und dessen Monomethyl- und Monoethylether sowie Dipropylenglykol und dessen Monomethyl- und Monoethylether können gegebenenfalls anwesend sein, beispielsweise in einer Menge zwischen 0,5 und 30, vorzugsweise etwa 1 bis etwa 15, insbesondere etwa 5 bis etwa 10 Gew.-% der Färbemittelzusammensetzung.

Diese Mittel können auch Pflegemittel wie Öle und Fette in geringen Mengen enthalten.
Solche sind beispielsweise Sonnenblumenöl, Mandelöl, Pfirsichkernöl, Weizenkeimöl, Macadamianußöl, Nachtkerzenöl, Jojobaöl, Ricinusöl, oder auch Oliven- bzw. Sojaöl, Lanolin und dessen Derivate, ebenso Mineralöle wie Paraffinöl und Vaseline.
Synthetische Öle und Wachse sind beispielsweise Silikonöle, Polyethylenglykole, etc.
Weitere geeignete hydrophobe Komponenten sind insbesondere Fettsäureester wie isopropylmyristat, -palmitat, -stearat und -isostearat, Oleyloleat, Isocetylstearat, Hexallaurat, Dibutyladipat, Dioctyladipat, Myristylmyristat, Oleylerucat, Polyethylenglykol- und Polyglycerylfettsäureester wie PEG-7-glycerylcocoat, Cetylpalmitat, etc.

Die erfindungsgemäßen Zusammensetzungen enthalten vorzugsweise ein Verdickungsmittel.
Als solche sind insbesondere Cellulosederivate wie Hydroxyalkylcellulosen, Xanthan Gum, Guar Gum, sowie synthetische Polymere, insbesondere Acrylsäure-Polymere, geeignet.

Ihre Menge ist abhängig von der gewünschten Viskosität und den sonstigen anwesenden Bestandteilen, insbesondere den Polymeren.

Die Viskosität der erfindungsgemäßen Mittel liegt bei 1000 bis 50 000, insbesondere etwa 1500 bis 30 000, vor allem etwa 2 500 bis 25 000 mPa.s bei 25°C, gemessen im Brookfield-Rotationsviskosimeter mit einer Spindel Nr. 5 bei 5rpm.

Der pH-Wert liegt vorzugsweise im Bereich zwischen etwa 2 und 7,5, vorzugsweise 2,5 und 6,5, insbesondere etwa 3,5 und 5,5.
Geeignete transparente Verpackungsmaterialien sind dem Fachmann bekannt, z.B. Glas oder, besonders bevorzugt, transparente Kunststoffe wie Polyethylen, Polypropylen, etc.

Die folgenden Beispiele dienen der Illustration der Erfindung.

### Beispiele

| **Bestandteile** | | **Nr.1** | **Nr.2** | **Nr.3** | **Nr.4** | |
|---|---|---|---|---|---|---|
| Hydroxyethylcellulose | | 0,70 | 1,50 | 0,50 | - | |
| Hydroxypropylmethylcellulose | | - | - | 0,50 | 1,00 | |
| Polyquaternium -10 | | 0,40 | 0,80 | 0,50 | - | |
| Polyquaternium-4 | | - | - | - | 0,50 | |
| Benzophenone-4 | | 0,10 | 0,10 | 0,10 | 0,10 | |
| Cetrimoniumchlorid | | 0,25 | 0,50 | 1,00 | - | |
| Stearyltrimethylammoniumchlorid | | 0,25 | - | - | 0,75 | |
| Ethanol | | 30,00 | 20,00 | - | 30,00 | |
| Isopropylalkohol | | - | - | 25,00 | - | |
| Vinylpyrrolidon/Vinylacetat-Copolymerisat | | 0,50 | 0,60 | 0,50 | 0,50 | |
| 1,2-Propandiol | | 0,30 | 0,30 | 0,30 | 0,30 | |
| Panthenol | | 0,20 | 0,20 | 0,20 | 0,20 | |
| PEG-60 Hydriertes Ricinusöl | | 0,25 | 0,30 | 0,50 | 0,40 | |
| Parfum | | 0,30 | 0,30 | 0,30 | 0,30 | |
| Basic Yellow 57 | | 0,005 | - | - | - | |
| Basic Brown 16 | | 0,002 | - | - | - | |
| Basic Blue 99 | | - | 0,01 | - | 0,005 | |
| Basic Red 76 | | - | - | 0,05 | - | |
| HC-Red 1 | | - | - | 0,01 | - | |
| Basic Violet 14 | | - | - | - | 0,001 | |
| Wasser | ad | 100,00 | 100,00 | 100,00 | 100,00 | Gew.-% |
| Erzielte Haarfärbung | | Gold-farben | Braun | Rot | Silber | |
| Viskosität bei 25°C (Brookfield, Spindel Nr.5) | | 1300 | 12000 | 6500 | 4000 | mPa.S |

Die Zusammensetzungen wurden in transparente Polyethylentuben abgepackt und einem viermonatigen Lagertest bei 40°C unterzogen.
Es trat keinerlei Instabilität auf; die Produkte unterschieden sich in ihrer Transparenz und ihrem Verhalten nicht vom Ausgangsprodukt.

In einem Färbetest wurden die Zusammensetzungen auf nasses Menschenhaar aufgebracht, nach etwa zwanzigminütiger Einwirkung gewaschen und getrocknet.
Es wurde ein glänzend gefärbtes Haar, das gut kämmbar war und einen lockeren, vollen Griff aufwies, erhalten.
Die Färbung blieb über fünf Haarwäschen stabil

### Beispiel 5

Es wurde ein weiteres transparentes Farbgel der folgenden Zusammensetzung hergestellt, das anionische direktziehende Farbstoffe enthielt.

| | |
|---|---|
| Dimethicone Copolyol | 1,50 (Gew.-%) |
| Cetrimoniumchlorid | 0,80 |
| Ethanol | 15,00 |
| Polyvinylpyrrolidon | 0,50 |
| Propylencarbonat | 15,00 |
| Milchsäure, 90%-ig | 5,00 |
| Natriumhydroxid, 32%-ig | 0,20 |
| Polyquatemium -6 | 0,60 |
| Quaternäres Dimethylaminoethylmethacrylat- Homopolymerisat (Polyquaternium-37;50%-ig in einem Propylenglykoldicaprat-dicaprylat / PPG-1-Trideceth-6 Gemisch; Salcare^{R}SC96) | 3,50 |
| Acid Orange 7 (C.I.-No. 15510) | 0,15 |
| Acid Yellow 3 (C.I.-No. 47005) | 0,10 |
| Acid Violet 43 (C.I.-No. 60 730) | 0,25 |
| Wasser | ad 100,00 |
| pH-Wert: | 3,00 |
| Viskosität bei 20°C im Rotationsviskosimeter nach Brookfield | |
| (Spindel Nr. 5, 5rpm): | ∼33 000 mPa.s |

Die Zusammensetzung wurde in transparente Polyethylentuben abgefüllt und einem viermonatigen Lagertest bei 40°C unterzogen.

Es trat keinerlei Instabilität oder Ausfällung auf, das Produkt unterschied sich nicht vom Ausgangsprodukt.

Die Zusammensetzung wurde auf nasses Haar aufgebracht.
Nach etwa zwanzigminütiger Einwirkung wurde gewaschen, gespült und getrocknet.
Es wurde ein glänzend mittelbraun gefärbtes Haar, das leicht kämmbar war, einen vollen Griff und Spannkraft aufwies, erhalten. Die Färbung blieb über fünf Haarwäschen haltbar.

## Patentansprüche

1. Gelförmiges Mittel zum Färben und Tönen von menschlichen Haaren, enthaltend
a) 0,001 bis 5 Gew.-% mindestens eines direktziehenden Haarfarbstoffs:
b) 0,1 bis 10 Gew.-% mindestens einer langkettigen quaternären Ammoniumverbindung ausgewählt aus Cetyltrimethylammoniumchlorid, Dimethyldiethylammoniumchlorid, Trimethylcetylammoniumbromid, Stearyltrimethylammoniumchlorid, Dimethylstearylbenzylammoniumchlorid, Benzyltetradecyldimethylammoniumchlorid, Dimethyldihydriertes-Talgammoniumchlorid, Laurylpyridiniumchlorid, Lauryldimethylbenzylammoniumchlorid, Behenyltrimethylammoniumchlorid, Lauryltrimethylammoniumchlorid, Tris-(oligooxyethyl)alkylammoniumphosphat, Cetylpyridiniumchlorid, Esterquats der allgemeinen Formel (I) 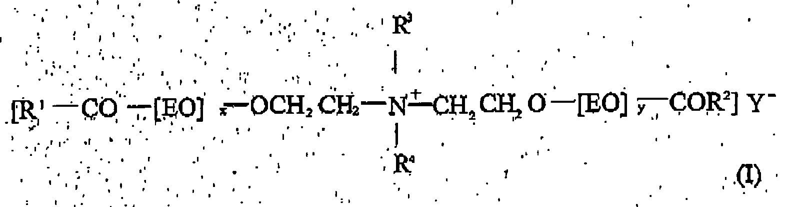 in der R¹ und R² für eine gegebenenfalls hydroxysubstituierte C₈-C₂₂-Alkyl- oder Alkenylgruppe, R³ und R⁴ für eine C₁-C₃-Alkylgruppe oder eine Gruppe -CH₂-CH₂-O-[EO]_{z}H sowie x,y und z für 0 bis 5 und Y für ein Anion stehen,> und Amidoquats der allgemeinen Formel (II) 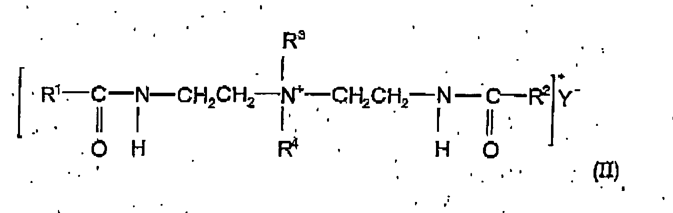 in der R¹ und R² jeweils für eine gegebenenfalls hydroxysubstituierte C₈-C₂₂-Alkyl- oder Alkenylgruppe, R³ und R⁴ für eine C₁-C₃-Alkylgruppe oder eine Gruppe-CH₂-CH₂-O-[EO]ₓ-H, sowie x für 0 bis 5, Y⁻ für ein Anion stehen;
c) 0,1 bis 5 Gew-% mindestens eines kationischen Polymeren;
d) 0,1 bis 5 Gew.-% mindestens eines nichtionischen und/oder amphoteren bzw. zwitterionischen Polymeren;
e) 15 bis 50 Gew.-% mindestens eines niederen Alkohols, ausgewählt aus Ethanol, n-Propanol und/oder Isopropylalkohol; und
f) Wasser,
das eine Viskosität von 1000 bis 50 000 MPa.s bei 25°C, gemessen im Brookfield-Viskosimeter RVT, Spindel Nr. 5 aufweist.

2. Mittel nach Anspruch 1, **dadurch gekennzeichnet, daß** es eine Viskosität von 1 500 bis 25 000 mPa.s, bei 25°C, gemessen im Brookfield-Viskosimeter RVT, Spindel Nr. 5, aufweist.

3. Mittel nach Anspruch 1 und/oder 2, **dadurch gekennzeichnet, daß** es in einem transparenten Behältnis abgepackt ist.

## Claims

1. Composition in form of a gel for the permanent and semi-permanent coloration of human hair, comprising
a) 0.001 % to 5 % by weight of at least one direct-acting hair dyestuff;
b) 0.1 % to 10 % by weight of at least one long-chain quaternary ammonium compound selected from cetyl trimethyl ammonium chloride, dimethyl diethyl ammonium chloride, trimethyl cetyl ammonium bromide, stearyl trimethyl ammonium chloride, dimethyl stearyl benzyl ammonium chloride, benzyl tetradecyl dimethyl ammonium chloride, dimethyl dihydrogenated tallow ammonium chloride, lauryl pyridinium chloride, lauryl dimethyl benzyl ammonium chloride, behenyl trimethyl ammonium chloride, lauryl trimethyl ammonium chloride, tris-(oligooxyethyl) alkyl ammonium phosphate, cetyl pyridinium chloride; esterquats of the general formula (I) 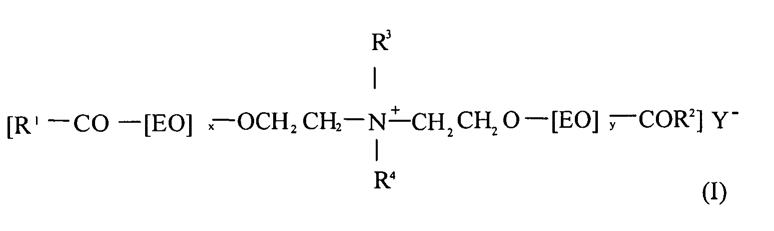 wherein R¹ and R² stand for an optionally hydroxy-substituted C₈-C₂₂-alkyl or alkenyl group, R³ and R⁴ stand for a C₁-C₃-alkyl group or a group -CH₂-CH₂-O-[EO]_{z}H, x, y and z stand for 0 to 5 and Y⁻ stands for an anion, and amidoquats of the general formula (II) 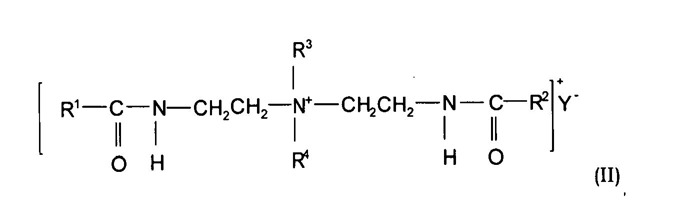 wherein R¹ and R² each stand for an optionally hydroxy-substituted C₈-C₂₂-alkyl or alkenyl group, R³ and R⁴ stand for a C₁-C₃-alkyl group or a group -CH₂-CH₂-O-[EO]ₓ-H, x stands for 0 to 5, and Y⁻ stands for an anion.
c) 0.1 % to 5 % by weight of at least one cationic polymer;
d) 0.1 % to 5 % by weight of at least one nonionic and/or amphoteric or zwitterionic polymer;
e) 15 % to 50 % by weight of at least one lower alcohol, selected from ethanol, n-propanol and/or isopropyl alcohol; and
f) water,
which has a viscosity of 1,000 to 50,000 mPa.s at 25°C, measured in a Brookfield viscosimeter RVT, no. 5 spindle.

2. Composition according to claim 1, **characterized in that** it has a viscosity of 1,500 to 25,000 mPa.s, at 25°C, measured in a Brookfield viscosimeter RVT, no. 5 spindle.

3. Composition according to claim 1 and/or 2, **characterized in that** it is packed into a transparent container.

## Revendications

1. Agent sous forme de gel pour teindre et nuancer des cheveux humains, contenant
a) 0,01 à 5 % en poids d'au moins un colorant capillaire montant directement sur les fibres ;
b) 0,1 à 10 % en poids d'au moins un composé d'ammonium quaternaire à longue chaîne choisi parmi le chlorure de cétyltriméthylammonium, le chlorure de diméthyldiéthylammonium, le bromure de triméthylcétylammonium, le chlorure de stéaryl-triméthylammonium, le chlorure de diméthylstéaryl-benzylammonium, le chlorure de benzyltétradécyl-diméthylammonium, le chlorure de diméthyl-(suif dihydrogéné)-ammonium, le chlorure de lauryl-pyridinium, le chlorure de lauryldiméthylbenzylammonium, le chlorure de béhényltriméthylammonium, le chlorure de lauryltriméthylammonium, le phosphate de tris-(oligo-oxyéthyl)alkylammonium, le chlorure de cétylpyridinium,
les esterquats de formule générale (I) 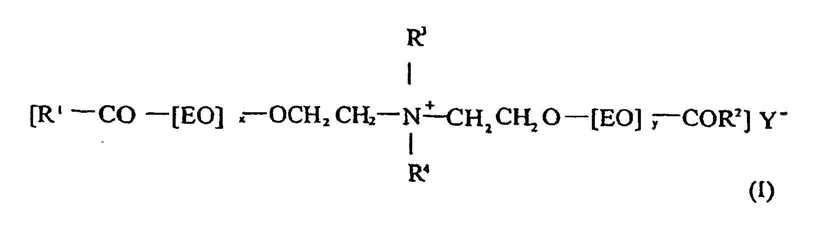 dans laquelle R¹ et R² représentent un groupe alkyle ou alcényle en C₈ à C₂₂, éventuellement à substitution hydroxy, R³ et R⁴ représentent un groupe alkyle en C₁ à C₅, ou un groupe -CH₂-CH₂-O-[EO]_{z}-H et x, y et z valent de 0 à 5 et Y⁻ représente un anion,
et les amidoquats de formule générale (II) 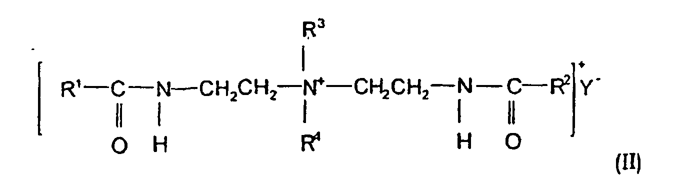 dans laquelle R¹ et R² représentent respectivement un groupe alkyle ou alcényle en C₈ à C₂₂, éventuellement à substitution hydroxy, R³ et R⁴ représentent un groupe alkyle en C₁ à C₃, ou un groupe -CH₂-CH₂-O-[EO]ₓ-H, et x vaut de 0 à 5, Y⁻ représente un anion ;
c) 0,1 à 5 % en poids d'au moins polymère cationique ;
d) 0, 1 à 5 % en poids d'au moins un polymère non ionique et/ou amphotère ou bien zwittérionique ;
e) 15 à 50 % en poids d'au moins un alcool inférieur, choisi parmi l'éthanol, le n-propanol, et/ou l'alcool isopropylique ; et
f) de l'eau,
qui présente une viscosité de 1 000 à 50 000 mPa.s à 25°C, mesurée dans un viscosimètre Brookfield RVT, broche n° 5.

2. Agent selon la revendication 1, **caractérisé en ce qu'**il présente une viscosité de 1 500 à 2 500 mPa.s à 25°C, mesurée dans un viscosimètre Brookfield RVT broche n° 5.

3. Agent selon la revendication 1 et/ou 2, **caractérisé en ce qu'**il est emballé dans un récipient transparent.
